(19) Europäisches Patentamt
European Patent Office
Office européen des brevets

(11) Veröffentlichungsnummer: **0 264 867**
**B1**

# (12) EUROPÄISCHE PATENTSCHRIFT

(45) Veröffentlichungstag der Patentschrift: **27.12.90**

(51) Int. Cl.⁵: **C07C 309/04,** E21B 43/22

(21) Anmeldenummer: **87115219.5**

(22) Anmeldetag: **17.10.87**

(54) **Styrylaryloxy-ethersulfonate, Verfahren zu deren Herstellung und deren Verwendung bei der Erdölförderung.**

(30) Priorität: **24.10.86 DE 3636277**

(43) Veröffentlichungstag der Anmeldung:
**27.04.88 Patentblatt 88/17**

(45) Bekanntmachung des Hinweises auf die Patenterteilung:
**27.12.90 Patentblatt 90/52**

(84) Benannte Vertragsstaaten:
**CH DE FR GB IT LI NL SE**

(56) Entgegenhaltungen:
**US-A- 4 318 816**

**HOUBEN-WEYL: "Methoden der organischen Chemie", Band IX, Schwefel-, Selen-, Tellurverbindungen, 4. Auflage, Herausgeber E. MÜLLER, Seiten 372-377, Georg Thieme Verlag, Stuttgart, DE**

(73) Patentinhaber: **HOECHST AKTIENGESELLSCHAFT, Postfach 80 03 20, D-6230 Frankfurt am Main 80(DE)**

(72) Erfinder: **Schmidt, Manfred, Dr., Mörike-Strasse 5, D-6233 Kelkheim (Taunus)(DE)**
Erfinder: **Rupp, Walter Dr., Am Eichkopf 6, D-6240 Königstein/Taunus(DE)**
Erfinder: **Schneider, Gerhart, Dr., Ziegelheck 3, D-6240 Königstein/Taunus(DE)**
Erfinder: **Kohn, Eva Maria, Parkstrasse 1, D-6232 Bad Soden am Taunus(DE)**

EP 0 264 867 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Styrylaryloxy-ethersulfonate, deren Herstellung und Verwendung als Tenside, insbesondere für die tertiäre Erdölgewinnung sowie synergistische Tensidkombinationen.

Bei der Gewinnung von Öl aus unterirdischen Lagerstätten gelingt es im allgemeinen nur 20 - 30 % des ursprünglich vorhandenen Öls durch primäre Gewinnungsverfahren zu fördern. Hierbei gelangt das Öl mit Hilfe des natürlichen Lagerstättendrucks an die Erdoberfläche. Bei der sekundären Gewinnung wird Wasser in die geologische Formation eingepreßt und das Öl durch mehrere Produktionssonden gefördert. Dieses Wasserfluten als Sekundärmaßnahme ist relativ billig und wird daher häufig verwendet, führt jedoch in vielen Fällen nur zu einer geringen Mehrentölung der Lagerstätte.

Nach Beendigung der sekundären Erdölfördermaßnahmen ist allein durch Zufuhr mechanischer Energie eine weitere wirtschaftliche Erdölgewinnung zu erreichen. In dem heterogenen Porenraum überholt das niedriger viskose Wasser das höher viskose Öl, so daß fast nur noch Wasser und kein Öl mehr gefördert wird. Hat der Verwässerungsgrad bei ca. 98 % die Wirtschaftlichkeitsgrenze überschritten, so kommen nur noch Verfahren der tertiären Erdölgewinnung in Frage. Darunter versteht man Verfahren, bei denen entweder die Viskosität des Öls erniedrigt und/oder die Grenzflächenspannung zwischen Wasser und Öl erniedrigt wird.

Die meisten Verfahren lassen sich als thermische Ölgewinnungsverfahren, Lösungs- oder Mischfluten, Tensid-oder Polymerfluten bzw. als Kombinationen von mehreren der genannten Verfahren einordnen. Bei thermischen Gewinnungsverfahren wird Dampf oder heißes Wasser in die Lagerstätte injiziert oder es erfolgt eine in situ Verbrennung. Beim Lösungs- oder Mischungsverfahren wird Lösemittel für das Erdöl (Gas oder eine Flüssigkeit) in die Lagerstätte injiziert.

Tensidflutverfahren beruhen auf einer starken Erniedrigung der Grenzflächenspannung zwischen Öl- und Flutwasser. Je nach Tensidkonzentration unterscheidet man Tensidfluten (low tension flooding), micellares Fluten und Emulsionsfluten.

In der Monographie von D.O. Shah und R.S. Schechter: Improved Oil Recovery by Surfactant and Polymerflooding, Academic Press Inc., New York, sowie in zahlreichen Patentschriften werden eine Vielzahl von Tensiden aufgeführt, die beim Tensidflutprozeß Verwendung finden können. Als Tenside werden dabei vor allem Sulfonate, wie z.B. synthetische und natürliche Petrolsulfonate, Alkylsulfonate, wie z.B. $C_{13}$-$C_{18}$-sec-Alkansulfonat-Na MG 328/350, $\alpha$-Olefinsulfonat-Na sowie (Vinyliden)-Olefinsulfonat-Na, Alkylarlysulfonat, wie z.B. Dodecylbenzolsulfonat-Na, Alkyltoluolsulfonate oder Alkylxylolsulfonate beschrieben. Diese Sulfonate besitzen aber nur eine sehr niedrige Toleranzgrenze gegenüber dem Salzgehalt der Lagerstättengewässer. So sind z.B. Petrolsulfonate nur in einem Wasser mit einem Salzgehalt von 1,5 % NaCl löslich.

Sulfonate sind vor allem auch gegen die im Lagerstättenwasser enthaltenen Erdalkalien sehr empfindlich. Bei höheren Salzkonzentrationen bilden sich beim Einsatz dieser Tenside Fällungsprodukte, die zur Verstopfung des porösen Raumes der Formation führen können. Viele Lagerstätten besitzen höhere Salinitäten, z.B. in Norddeutschland 25 %. Um in diesen Lagerstättensystemen mit Sulfonaten arbeiten zu können, werden Kombinationen mit Alkoholen und/oder nichtionischen Tensiden (Alkyl- bzw. Alkylarylpolyglykolether) vorgeschlagen, die bei diesen Salzkonzentrationen stabil sind, aber meist wurde die ölmobilisierende Wirkung verschlechtert.

In der US P 3 827 497 wird die Verwendung der Salze sulfatierter oxyalkylierter Alkohole vorgeschlagen. Es hat sich aber gezeigt, daß Salze, die die COS-Bindung enthalten, bei erhöhten Temperaturen hydrolytisch gespalten werden. In den US-Patenten 4 018 278, 4 088 189, 4 077 471, 4 120 228, 4 318 816, 4 194 564, 3 977 471 werden Ethersulfonate ($C_{12}$-$C_{15}$-Alkyl- und Alkylarylethersulfonate) beschrieben, die in Lagerstättenwasser mit hohem Salzgehalt (200 g/l) und bei einer Lagerstättentemperatur bis zu 120°C beständig sind. Mit Hilfe der Tenside kann aber nur bei ausgewählten Lagerstätten eine ölmobilisierende Wirksamkeit festgestellt werden. Ähnliche Erfahrungen wurden mit den in der DOS 2 724 442 und DOS 2 724 490 beschriebenen Produkten Alkyletherpropansulfonat- bzw. Alkyletherglycidylethersulfonat gemacht. Bekannt ist auch die Verwendung von Tributylphenoletherglycidylsulfonaten und Tributylethersulfonaten bei der Erdölförderung (DE-OS 33 47 578 und DE-OS 33 46 676).

Die einzelnen Lagerstätten unterscheiden sich in der Temperatur, die zwischen 20 und 120°C liegen kann, in der Öl-Zusammensetzung, das paraffinisch, naphthenisch, aromatisch, hoch-, mittel- oder niedrigviskos sein kann, im Salzgehalt und der Salzzusammensetzung, der zwischen 3 % und 25 % NaCl mit unterschiedlichem Gehalt an Erdalkalien liegen kann, in der Lagerstättenformation, der mineralogischen Zusammensetzung (z.B. Sandstein oder Kalkstein), Porosität und Permeabilität. Es ist daher das Ziel, Tenside bzw. Tensidkombinationen zu finden, die bei möglichst allen vorhandenen Lagerstättenbedingungen (d.h. im Temperaturbereich von 20 bis 120°C, Salzgehalt 30 bis 250 g/l) und bei den verschiedenen Öltypen wirksam sind.

Es wurde nun gefunden, daß Styryl-phenol- bzw. kresolethersulfonate bei der Verwendung als Tenside für die Erdölförderung bei unterschiedlichen Lagerstättenparametern wirksam sind.

Gegenstand der Erfindung sind Styrylaryloxy-ethersulfonate der Formel

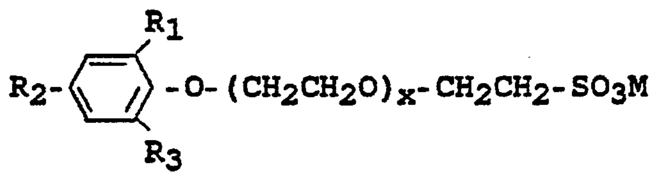

wobei entweder $R_1$ Styryl bedeutet und gleichzeitig $R_2$ und $R_3$ gleich oder verschieden sind und Wasserstoff oder Styryl bedeuten oder $R_1$ und $R_2$ ungleich sind und jeweils Methyl oder Styryl bedeuten und gleichzeitig $R_3$ Wasserstoff oder Styryl bedeutet, x eine Zahl von 2 bis 20, vorzugsweise 2 bis 12 und M ein Ammonium- oder Alkalimetallkation bedeutet.

Bevorzugt sind die Styrylkresol-ethersulfonate unter der obigen Formel, worin $R_1$ und $R_2$ ungleich sind und jeweils Methyl oder Styryl und $R_3$ Wasserstoff oder Styryl bedeuten und x und M die obengenannten Bedeutungen haben. Unter dem Styryl-Rest ist in allen Fällen eine Gruppe der Formel $C_6H_5$-CH-$CH_3$ zu verstehen.

Diese Verbindungen werden hergestellt gemäß dem folgenden Reaktionsschema:

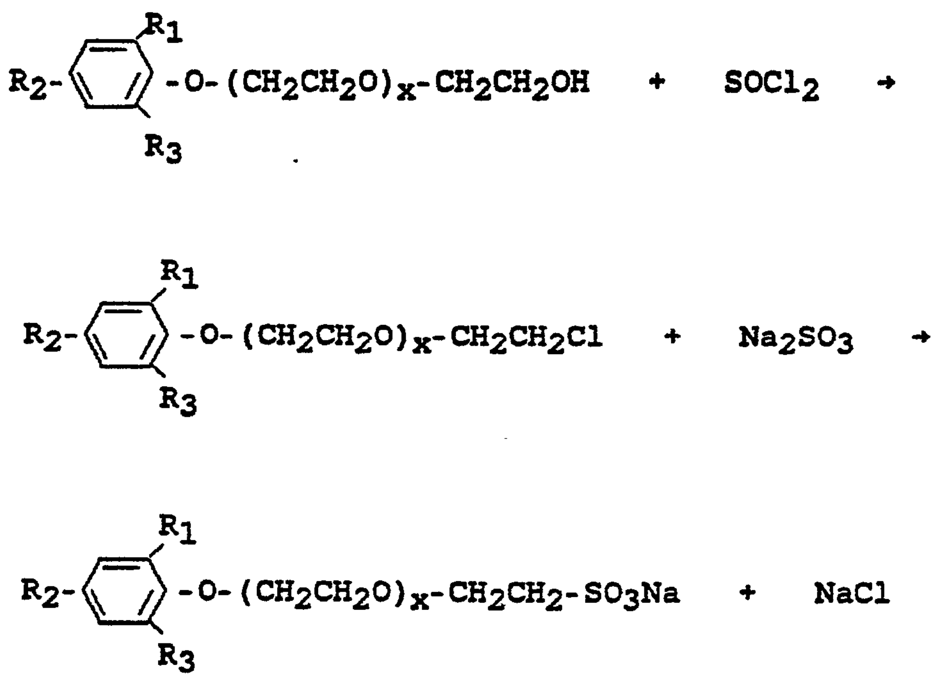


Die Umsetzung der Zwischenverbindung mit dem Natriumsulfit erfolgt bei 130 bis 190°C, vorzugsweise bei 160 - 180°C in wäßriger Lösung, gegebenenfalls zusammen mit Cosolventien wie niederen Alkoholen (iso-Propanol, Butanol), Glykolen oder Glykolethern, Zur Erhöhung der Ausbeute ist es von Vorteil, dem reaktionsansatz eine geringe Menge an Endprodukt aus einem vorherigen Ansatz zuzugeben, Anstelle von Natriumsulfit kann auch Kaliumsulfit genommen werden.

Die Mono- und Di-Styrylphenol- und -Kresol-ethersulfonate der obigen Formel sind Tenside, die sich durch Beständigkeit in einem weiten Temperatur- und pH-Bereich auszeichnen. Wäßrige Lösungen dieser Verbindungen erniedrigen die Oberflächenspannung an der Phasengrenze Tensidlösung/Luft auf Werte in der Größenordnung von ca. 40 $mNm^{-1}$ (nach Du Nouy) und die Grenzflächenspannung Öl/Tensidlösung auf Werte von ca. $10^{-2}$ $mNm^{-1}$ (Spinning-drop-interfacial tensiometer). Aufgrund ihrer Wirksamkeit und Stabilität über einen weiten pH-Bereich eignen sich diese Verbindungen für alkalische und für saure Metallreinigungsmittel sowie Feuerlöschmittel. Sie sind außerdem wirksame Emulgiermittel für die Emulsions-Polymerisation und eignen sich als Stabilisatoren für Latex und andere Polymer-Emulsionen. Von besonderem Interesse i st auch die Verwendung dieser Verbindungen bei der tertiären Erdölförderung und bei der Sondenstimulierung und Frac-Behandlung von Erdöl-Lagerstätten. Der Einsatz der Verbindungen gemäß der vorliegenden Erfindung erhöht bei diesem Verfahren die Ölausbeute. In allen Fällen wird das Tensid im allgemeinen in Mengen von 0,01 bis 10, vorzugsweise 0,05 bis 3 % eingesetzt.

Die erfindungsgemäßen Styryl-phenol- und -kresol-ethersulfonate können besonders vorteilhaft in Kombination mit anderen anionischen Tensiden, wie z.B. Olefinsulfonate, Alkansulfonate, α-Olefinsulfonate, interne Olefinsulfonate, Alkylarylsulfonate, Dodecylbenzolsulfonate, Petrolsulfonate, Alkylxylolsulfonate, Alkyltoluolsulfonate und nichtionischen Tensiden von Alkyl- bzw. Alkylphenylpolyglykolethern eingesetzt werden. Von besonderem Interesse sind dabei Mischungen aus den erfindungsgemäßen Mono- bzw. Di-Styryl-kresol-ethersulfonaten und Alkansulfonaten bzw. Olefinsulfonaten und/oder Petrolsulfonaten im Mischungsverhältnis von 4:1 bis 1:4. Als weitere Zusätze kommen Alkohole, Glykolether, Alkylglykolether und Dialkylglykolether in Frage. Die Viskosität des Flutwassers kann außerdem durch Polymere wie z.B. Hydroxyethylcellulose, Polyacrylamide, Copolymere auf Basis Acrylamid oder Polysaccharide erhöht werden.

**Beispiel 1**

Mono-Styryl-o-kresol(6 EO)-sulfonat-Na
(α-Phenylethyl-o-kresol(6 EO)-sulfonat-Na)

120 g (0,245 Mol) des aus α-Phenylethyl-o-kresol-(6 EO)-polyglykolether durch OH/Cl-Austausch mittels Thionylchlorid hergestellten Chlorids (organisch gebundenes Chlor 7,25 %) werden mit 37 g Natriumsulfit, 230 g Wasser, 5 g iso-Propanol, 50 g Sulfonatlösung aus einem vorhergehenden Ansatz und ggf. NaOH bis zum pH-Wert 9-10 in einen 0,7 l Rührautoklaven gefüllt und 6 Stunden bei 175° C gerührt. Das erhaltene Produkt enthält 27,9 % Sulfonat, was einer Ausbeute von 80,0 % (bezogen auf das eingesetzte Chlorid) und einem Sulfonat-Molgewicht von 558 entspricht.

**Beispiel 2**

Mono-styryl-p-kresol-8 EO-sulfonat-Na

140 g (0,25 Mol) des aus α-Phenylethyl-p-kresol-(8 EO)-polyglykolether durch OH/Cl-Austausch mittels Thionylchlorid hergestellten Chlorids (organisch gebundenes Chlor 6,35 %) werden mit 36 g Natriumsulfit, 260 g Wasser, 5 g iso-Propanol, 50 g Sulfonat-Lösung aus einem vorhergehenden Ansatz und ggf. NaOH bis zum pH 9-10 in einem 0,7 l Rührautoklaven gefüllt und 6 Stunden bei 175°C gerührt. Das erhaltene Produkt enthält 28,0 % Sulfonat, was einer Ausbeute von 79 % (bezogen auf das eingesetzte Chlorid) und einem Sulfonat-Molgewicht von 627 entspricht.

**Beispiel 3**

Di-Styryl-o-kresol-13 EO-sulfonat-Na
(Di-α-phenylethyl-o-kresol-13 Eo-sulfonat-Na)

140 g (0,155 Mol) Di-α-phenyl-o-kresol-13 EO-chlorid (organisch gebundenes Chlor 3,9 %) werden mit 24 g Natriumsulfit, 280 g Wasser, 5 g iso-Propanol, 50 g Sulfonat-Lösung aus einem vorhergehenden Ansatz und ggf. NaOH bis zum pH 9-10 in einen 0,7 l Rührautoklaven gefüllt und 6 Stunden bei 175°C gerührt. Das erhaltene Produkt enthält 27,6 % Sulfonat, was einer Ausbeute von 81,6 % (bezogen auf das eingesetzte chlorid) und ein Sulfonat-Molgewicht von 974 entspricht.

**Beispiel 4**

Di-Styryl-p-kresol-17 EO-sulfonat-Na
(α-Phenylethyl-p-kresol-17 EO-sulfonat-Na)

120 g (0,1 Mol) Di-α-phenylethyl-p-kresol-17 EO-chlorid (organisch gebundenes Chlor 3,3 %) werden mit 17 g Natriumsulfit, 200 g Wasser, 5 g iso-Propanol, 50 g Sulfonat-Lösung aus einem vorhergehenden Ansatz und ggf. NaOH bis zum pH 9-10 in einen 0,7 l Rührautoklaven gefüllt und 6 Stunden bei 175°C gerührt. Das erhaltene Produkt enthält 28,8 % Sulfonat, was einer Ausbeute von 77,8 % (bezogen auf das eingesetzte Chlorid und ein Sulfonat-Molgewicht von 1150 entspricht.

Zur Bestimmung der Wirksamkeit der erfindungsgemäßen Verbindungen und synergistischen Tensidkombination wird die in der US-Patentschrift 4 008 165 beschriebene Mikrokapillarentölungs-Methode, die Bestimmung der Grenzflächenspannung nach der Spinning-drop-interfacial-Tensiometer-Methode, das Phasenverhalten nach Winsor und Laborflutversuche in mit Sand gefüllten Glasrohren herangezogen.

Bei der Mikrokapillarentölung werden als Modell für den Porenraum der Lagersätte Mikrokapillaren

aus glas der Fa. Drummond Scientific Co./USA verwendet, die bei einem Volumen von 5 µl eine Länge von 30 mm und einen Durchmesser von 0,45 mm aufwiesen.

Die Mikrokapillaren werden an einem Ende abgeschmolzen, in einem Exsikator evakuiert und mit Rohöl gefüllt. Die Kapillaren werden in Tensidlösungen (Reagenzgläser), die im wasserbad temperiert werden, mit der Öffnung nach oben senkrecht eingebracht und die Verdrängung des Öls wird in Abhängigkeit von der Zeit visuell registriert.

Mit Hilfe des folgenden Beurteilungsschemas kann die Wirksamkeit der Tenside in Abhängigkeit von deren Konzentration, der Salzkonzentration, pH-Wert, Temperatur und Ölzusammensetzung bestimmt werden.

**Wert**

9 leer (30 mm) nach 10 Minuten
8 leer nach 1 Stunde
7 leer nach 3 Stunden
6 leer nach 20 Stunden
5 16 - 25 mm Entleerung nach 20 Stunden
4 9 - 15 mm Entleerung nach 20 Stunden
3 4 - 8 mm Entleerung nach 20 Stunden
2 1 - 3 mm Entleerung nach 20 Stunden
1 Spur Entleerung nach 20 Stunden
0 unverändert nach 20 Stunden

Diese Methode bietet den Vorteil, daß bei dem geringen Durchmesser der Mikrokapillaren Viskosität und Dichte der Öle keinen großen Einfluß auf die Entölungswirkung ausüben und es möglich ist, mit Lagerstättenöl und Lagerstättenwasser zu arbeiten.

Nach Taber J. Petr. Techn. 3 (1969), S. 3 - 12 sind Tenside für die tertiäre Erdölgewinnung nur geeignet, wenn die Grenzflächenspannung an der Phasengrenze Öl/Salzwasser auf Werte kleiner $10^{-2}$ $mNm^{-1}$ erniedrigt wird. Für diese Bestimmung der Grenzflächenspannung an der Phasengrenze Öl/Wasser wird das von Wade und Burkowsky entwickelte Spinnning-drop-interfacial-tensiometer verwendet. (M. Burkowsky und C. Marx: Über den Mechanismus des Tensidflutens in hochsalinaren Systemen; Erdöl-Erdgas-Zeitschrift 95 (1979), S. 17 - 25).

Die Method beruht darauf, daß ein Öltropfen, der in eine um die horizontale Achse rotierende Kapillare gebracht wird, die eine Flüssigkeit (Salzwasser und Tensid) mit höherer Dichte enthält, deformiert wird. Der Tropfen wird gestreckt, bis ein Gleichgewicht der deformierenden Kräfte und der Grenzflächenspannung erreicht wird.

Die Grenzflächenspannung errechnet sich nach Vonnegut (B. Vonnegut, Rev. Sci. Instruments 13 (1942), S. 6 - 9) aus dem gemessenen Öltropfendurchmesser R, der Rotationsgeschwindigkeit W und dem Dichteunterschied Δd nach folgender Formel:

$$\gamma\ 1/2 = \frac{\Delta d \cdot W^2 \cdot R^3}{4}\ (mNm^{-1})$$

Nach dem heutigen Stand der Aufklärung des Mechanismus der Entölung beim Tensidfluten, ist die Ausbildung einer 3. Phase (Mittelphase) einer Mikroemulsion die Voraussetzung für ein optimales Tensidflutergebnis [Rieckmann, M., "Tertiäre Erdölgewinnung", Erdöl und Kohle - Erdgas-Petrochemie 36 (1983) 281-282), Healy, R.N. und Reed, R.L. Soc Petr. Eng. J. 10 (1979) 492-501, Obah, B. und Neumann, J.H. "über die Bildung von Mikroemulsionen", Tenside Detergents 20 (1983) 145 -151]. Diese gesuchte dritte Phase entsteht im System, wenn die Grenzflächenspannung an der Phasengrenze Öl/Salzwasser stark erniedrigt wird.

Bei der Ermittlung der Mittelphasen werden 5 ml Tensidlösung (mit Salzwasser) und 5 ml Öl (Lagerstättenöl oder Modellöl in ein Reagenzglas gebracht, das Reagenzglas zugeschmolzen kräftig geschüttelt und in einem Trockenschrank bei konstanter Temperatur gelagert. Nach einer Stunde Lagerzeit wird erneut kräftig geschüttelt und die Reagenzgläser werden dann ohne weitere Durchmischung gelagert. Nach einer Lagerzeit von 1 und 7 Tagen wird die Bildung der Phasen (Mittelphase) ermittelt.

Ein weiteres wichtiges Auswahlkriterium ist die ölmobilisierende Wirkung der Styrylethersulfonate, die in Laborflutversuchen untersucht wird, Geeignete Versuchsbedingungen sind Flutversuche mit künstlichen Schüttungen aus Sanden, Sandstein oder Kalkstein, die in Glasrohren eingefüllt sind. Bei der Durchführunhg der Versuche werden die Glasrohre der Fa. Quickfit (Länge: 15 cm, Innendurchmesser: 2,6 cm) mit Hilfe eines Vibrators Quarzsand bestimmter Korngrößen eingerüttelt. Das mit Sand gefüllte Flutrohr wird mit Fritte, Dichtung und Abschlußplatte versehen und auf Dichtigkeit geprüft. Die Rohre

werden mit entgastem Formationswasser gefüllt, die physikalischen Daten, Porosität und Permeabilität nach dem Darcy'schen Gesetz ermittelt und anschließend mit Öl getränkt. Die Rohre werden temperiert und nach Eichung der Druckaufnehmer und Überprüfung der Förderraten der Injektionspumpen kann Einpreßwasser injiziert werden. Der Beginn des Tensid- bzw. Polymerflutens setzt ein, wenn die Ölausbeute über einen längeren Zeitraum konstant bleibt (ca. 1,5 bis 2,0 PV). Anschließend an den Chemikalienslug, dessen Menge sich nach Konzentration, Viskosität Wirtschaftlichkeit usw. richtet, wird wieder Flutwasser injiziert. Beendet ist der Flutversuch, wenn kein bzw, nur noch sehr wenig Öl ausgeflutet wird. Die ausgebrachten (geförderten) Wasser- und Ölmengen werden volumetrisch bestimmt und grafisch gegen das Porenvolumen aufgetragen (Entölungskurve).

Die mit diesen Methoden gemessenen Werte sind in den folgenden Tabellen zusammengefaßt. In allen Fällen wurden 1 %ige wäßrige Lösungen der Tenside eingesetzt. (Ausnahmen sind angegeben). In allen Beispielen bedeutet "EO" Ethylenoxid. Das in den Beispielen benutzte Alkansulfonat hat in allen Fällen ein Molekulargewicht von 328, das Alkylxylolsulfonat hat ein Molekulargewicht von 390.

Beispiel 1

| | Oberflächenspannung nach Du Nouy ($mNm^{-1}$) (25°C / 40°C) | | | |
|---|---|---|---|---|
| | dest. Wasser (72,6/70,0 $mNm^{-1}$) 0,5 % | | 50 g/l Nacl (76/74 $mNm^{-1}$) 0,5 % | |
| | 25°C | 40°C | 25°C | 40°C |
| Mono-Styrylkresol (o) (6 EO) ethersulfonat-Na | 43,4 | 44,8 | 41,9 | 43,2 |
| Mono-Styrylkresol (o) (8 EO) ethersulfonat-Na | 45,8 | 45,8 | 43,3 | 43,8 |
| Di-Styrylkresol (o) (15 EO) ethersulfonat-Na | 46,4 | 46,2 | 43,7 | 44,2 |
| Mono-Styrylkresol (p) (8 EO) ethersulfonat-Na | 43,5 | 43,1 | 41,0 | 42,2 |
| Di-Styrylkresol (p) (17 EO) ethersulfonat-Na | 48,0 | 45,0 | 44,7 | 43,9 |

Beispiel 2

| | Grenzflächenspannung nach Du Nouy ($mNm^{-1}$) bei Raumtemperatur (RT), ph= 6,5, Gehalt an Wirksubstanz (WS)= 0,5 % | |
|---|---|---|
| | dest. Wasser/n-Octan (50,8/51,1 $mNm^{-1}$) | 50g/l NaCl/n-Octan (43,6 $mNm^{-1}$) |
| Mono-Styrylkresol (o) (6 EO) ethersulfonat-Na | 9,0 | 5,5 |
| Mono-Styrylkresol (o) (8 EO) ethersulfonat-Na | 9,0 | 6,0 |
| Di-Styrylkresol (o) (15 EO) ethersulfonat-Na | 8,4 | 6,3 |
| Mono-Styrylkresol (p) (8 EO) ethersulfonat-Na | 9,0 | 6,0 |
| Di-Styrylkresol (p) (17 EO) ethersulfonat-Na | 10,6 | 7,0 |

Beispiel 3

| | Grenzflächenspannung nach Du Nouy ($mNm^{-1}$) bei RT, pH 6,5, WS= 0,5 % | |
|---|---|---|
| | dest. Wasser/Toluol ($34,0/34,8\ mNm^{-1}$) | 50g/l NaCl/n-Octan ($33,3/33,5\ mNm^{-1}$) |
| Mono-Styrylkresol (o) (6 EO) ethersulfonat-Na | 4,9 | 1,5 |
| Mono-Styrylkresol (o) (8 EO) ethersulfonat-Na | 4,6 | 1,9 |
| Di-Styrylkresol (o) (15 EO) ethersulfonat-Na | 4,5 | 2,5 |
| Mono-Styrylkresol (p) (8 EO) ethersulfonat-Na | 4,5 | 1,6 |
| Di-Styrylkresol (p) (17 EO) ethersulfonat-Na | 5,0 | 2,4 |

Beispiel 4

| | Grenzflächenspannung nach Du Nouy ($mNm^{-1}$) bei 40°C, pH 6,5, WS= 0,5 % | |
|---|---|---|
| | dest. Wasser/Toluol ($32,5\ mNm^{-1}$) | 50g/l NaCl/n-Octan ($30,5\ mNm^{-1}$) |
| Mono-Styrylkresol (p) (8 EO) ethersulfonat-Na | 4,0 | 1,2 |
| Di-Styrylkresol (p) (17 EO) ethersulfonat-Na | 4,1 | 1,7 |

Beispiel 5

| | Grenzflächenspannung nach Du Nouy ($mNm^{-1}$) bei 40°C, pH 6,5, WS= 0,5 % | |
|---|---|---|
| | dest. Wasser/n-Octan ($29,8/40,4\ mNm^{-1}$) | 50g/l NaCl/n-Octan ($32,8\ mNm^{-1}$) |
| Mono-Styrylkresol (p) (8 EO) ethersulfonat-Na | 8,9 | 6,0 |
| Di-Styrylkresol (p) (17 EO) ethersulfonat-Na | 9,9 | 6,7 |

Beispiel 6

Mono-/Di-Styrylkresol-ethersulfonat-Na

| | NaCl/CaCl2 (g/l) | Temperatur | Löslichkeit | Grenzflächenspannung (Spinning-drop) | Mikrokapillarentölung | Phasenverhalten (Mittelphasenbildung) |
|---|---|---|---|---|---|---|
| | 140/60 | 25°C | + | ca.$10^{-2}$ $mNm^{-1}$ | 9 | + |
| Di-Styryl-o-kresol-6 EO-sulfonat Na | 150/0 | 40°C | + | ca.$10^{-2}$ $mNm^{-1}$ | 9 | + |
| Di-Styryl-o-kresol-13 EO-sulfonat Na | 180/20 | 40°C | + | ca.$10^{-2}$ $mNm^{-1}$ | 9 | + |
| Di-Styryl-o-kresol 8 EO sulfonat Na | 140/60 | 60°C | + | ca.$10^{-2}$ $mNm^{-1}$ | 8 | + |
| Di-Styryl-o-kresol-19 EO-sulfonat Na | 150/0 | 60°C | + | ca.$10^{-2}$ $mNm^{-1}$ | 9 | + |
| Paraffinöl | | | | | | |
| Di-Styryl-p-kresol-6 EO-sulfonat Na | 150/0 | 25°C | + | ca.$10^{-2}$ $mNm^{-1}$ | 8 | + |
| K-Öl | | | | | | |
| Di-Styryl-phenol-15 EO-sulfonat Na | 150/0 | 25°C | + | ca.$10^{-2}$ $mNm^{-1}$ | 6 | + |

Beispiel 7

| | | Grenzflächenspannung (Spinning drop) $mNm^{-1}$ Lagerstättenöl K, 35/15 g/l $NaCl/CaCl_2$ | | | |
|---|---|---|---|---|---|
| Di-Styryl-o-kresol-8 EO-sulfonat Na | Dodecylbenzol sulfonat-Na | 25°C | 40°C | 60°C | 80°C |
| 0,5 % | 0,5 % | $1 \cdot 10^{-2}$ | $1 \cdot 10^{-2}$ | $3 \cdot 10^{-2}$ | $4 \cdot 10^{-2}$ |
| 0 % | 1 % | * - | - | - | - |

* Lösungen nicht meßbar oder nicht wirksam

Beispiel 8

| | | Grenzflächenspannung (Spinning drop) $mNm^{-1}$ Lagerstättenöl K, 70/30 g/l $NaCl/CaCl_2$ | | | |
|---|---|---|---|---|---|
| Di-Styryl-o-kresol-8 EO-sulfonat Na | sec-Alkansulfonat-Na MG328 | 25°C | 40°C | 60°C | 80°C |
| 0,5 % | 0,5 % | $5 \cdot 10^{-2}$ | $2 \cdot 10^{-2}$ | $2 \cdot 10^{-2}$ | $4 \cdot 10^{-2}$ |
| 0 % | 1 % | * - | - | - | - |

* Lösungen nicht meßbar oder nicht wirksam

Beispiel 9

| | | Grenzflächenspannung (Spinning drop) $mNm^{-1}$ Lagerstättenöl K, 90/10 g/l $NaCl/CaCl_2$ | | | |
|---|---|---|---|---|---|
| Di-Styryl-o-kresol-8 EO-sulfonat Na | Dodecylbenzol-sulfonat-Na | 25°C | 40°C | 60°C | 80°C |
| 0,5 % | 0,5 % | $3 \cdot 10^{-2}$ | $2 \cdot 10^{-2}$ | $2 \cdot 10^{-2}$ | $5 \cdot 10^{-2}$ |
| 0 % | 1 % | * - | - | - | - |

* Lösungen nicht meßbar oder nicht wirksam

Beispiel 10

| | | Grenzflächenspannung (Spinning drop) $mNm^{-1}$ Lagerstättenöl K, 100 g/l NaCl, pH 6,5 | | | |
|---|---|---|---|---|---|
| Di-Styryl-o-kresol-8 EO-sulfonat Na | Dodecylbenzol-sulfonat-Na | 25°C | 40°C | 60°C | 80°C |
| 0,5 % | 0,5 % | $1 \cdot 10^{-2}$ | $4 \cdot 10^{-2}$ | $3 \cdot 10^{-2}$ | $3 \cdot 10^{-2}$ |
| 0 % | 1 % | * - | - | - | - |

* Lösungen nicht meßbar oder nicht wirksam

Beispiel 11

| | | Grenzflächenspannung (Spinning drop) $mNm^{-1}$ Lagerstättenöl K, 90/10 g/l $NaCl/CaCl_2$ | | | |
|---|---|---|---|---|---|
| Di-Styryl-o-kresol-13 EO-sulfonat Na | Dodecylbenzol-sulfonat-Na | 25°C | 40°C | 60°C | 80°C |
| 0,5 % | 0,5 % | $4 \cdot 10^{-2}$ | $9 \cdot 10^{-2}$ | $2 \cdot 10^{-2}$ | $7 \cdot 10^{-2}$ |
| 0 % | 1 % | *- | - | - | - |

* Lösungen nicht meßbar oder nicht wirksam

Beispiel 12

| | | Grenzflächenspannung (Spinning drop) $mNm^{-1}$ Lagerstättenöl I, 200 g/l NaCl | | | |
|---|---|---|---|---|---|
| Di-Styryl-p-kresol-6 EO-sulfonat Na | Dodecylbenzol-sulfonat-Na | 25°C | 40°C | 60°C | 80°C |
| 0,5 % | 0,5 % | $1 \cdot 10^{-2}$ (+) | $6 \cdot 10^{-2}$ (+) | $7 \cdot 10^{-2}$ (+) | $2 \cdot 10^{-2}$ (+) |
| 0 % | 1 % | *- | - | - | - |

(+) bedeutet die Bildung einer Mittelphase

* Lösungen nicht meßbar oder nicht wirksam

Beispiel 13

| | | Grenzflächenspannung (Spinning drop) $mNm^{-1}$ Lagerstättenöl K, 140/60 g/l $NaCl/CaCl_2$ | | | |
|---|---|---|---|---|---|
| Di-Styryl-o-kresol-6 EO-sulfonat Na | Dodecylbenzol-sulfonat-Na | 25°C | 40°C | 60°C | 80°C |
| 0,5 % | 0,5 % | ca. $10^{-2}$ | ca. $10^{-2}$ | ca. $10^{-2}$ | ca. $10^{-2}$ |
| 0 % | 1 % | *- | - | - | - |

* Lösungen nicht meßbar oder nicht wirksam

Beispiel 14

| | | Grenzflächenspannung (Spinning drop) $mNm^{-1}$ Lagerstättenöl K, 70/30 g/l $NaCl/CaCl_2$ | | | |
|---|---|---|---|---|---|
| Di-Styryl-o-kresol-13 EO-sulfonat Na | Dodecylbenzol-sulfonat-Na | 25°C | 40°C | 60°C | 80°C |
| 0,5 % | 0,5 % | $1 \cdot 10^{-2}$ | $1 \cdot 10^{-2}$ | $3 \cdot 10^{-2}$ | $8 \cdot 10^{-2}$ |
| 0 % | 1 % | *- | - | - | - |

* Lösungen nicht meßbar oder nicht wirksam

Beispiel 15

| | | Grenzflächenspannung (Spinning drop) $mNm^{-1}$ Lagerstättenöl K, 200 g/l NaCl/l | | | |
|---|---|---|---|---|---|
| Di-Styryl-o-kresol-6 EO-sulfonat Na | sec Alkan-sulfonat-Na | 25°C | 40°C | 60°C | 80°C |
| 0,5 % | 0,5 % | $1 \cdot 10^{-2}$ | $3 \cdot 10^{-2}$ | $1 \cdot 10^{-2}$ | $2 \cdot 10^{-2}$ |
| 0 % | 1 % | *- | - | - | - |

* Lösungen nicht meßbar oder nicht wirksam

Beispiel 16

| | | Grenzflächenspannung (Spinning drop) $mNm^{-1}$ Lagerstättenöl II, 150 g NaCl/l | | | |
|---|---|---|---|---|---|
| Di-Styryl-o-kresol-6 EO-sulfonat Na | Dodecylbenzol-sulfonat-Na | 25°C | 40°C | 60°C | 80°C |
| 0,5 % | 0,5 % | $2 \cdot 10^{-2}$ (6/+) | $3 \cdot 10^{-}$ (6/+) | $2 \cdot 10^{-2}$ (6/+) | $3 \cdot 10^{-3}$ (9/+) |
| 0 % | 1 % | *- | - | - | - |

Die Zahlen 6 bzw. 9 geben die bei der Mikrokapillarentölung gemessenen Werte an; (+) bedeutet die Bildung einer Mittelphase

* Lösungen nicht meßbar oder nicht wirksam

Beispiel 17

| | | Grenzflächenspannung (Spinning drop) $mNm^{-1}$ Lagerstättenöl I, 60°C | |
|---|---|---|---|
| Di-Styryl-o-kresol-18 EO-sulfonat Na | sec Alkan-sulfonat-Na | 180/20 g/l $NaCl/CaCl_2$ | 140/60 g/l $NaCl/CaCl_2$ |
| 0,5 % | 0,5 % | $6 \cdot 10^{-2}$ | $4 \cdot 10^{-2}$ |
| 0 % | 1 % | *- | - |

* Lösungen nicht meßbar oder nicht wirksam

Beispiel 18

| | | Grenzflächenspannung (Spinning drop) $mNm^{-1}$ Lagerstättenöl II, 135/15 g/l $NaCl/CaCl_2$ | | | |
|---|---|---|---|---|---|
| Di-Styryl-o-kresol-6 EO-sulfonat Na | sec Alkan-sulfonat-Na | 25°C | 40°C | 60°C | 80°C |
| 0,5 % | 0,5 % | $1 \cdot 10^{-2}$ | $2 \cdot 10^{-1}$ (2/–) | $5 \cdot 10^{-2}$ (0/+) | $3 \cdot 10^{-3}$ (9/+) |
| 0 % | 1 % | *- | - | - | - |

Die Zahlen 0,2 oder 9 geben die bei der Mikrokapillarentölung gemessenen Werte an; (+) bzw. (–) bedeuten, daß sich eine Mittelphase gebildet hat bzw. nicht gebildet hat.

* Lösungen nicht meßbar oder nicht wirksam

Beispiel 19

| | | Grenzflächenspannung (Spinning drop) $mNm^{-1}$ Lagerstättenöl I,200 g/l NaCl | | |
|---|---|---|---|---|
| Di-Styryl-p-kresol-11 EO-EO-sulfonat Na | sec Alkan-sulfonat-Na | 40°C | 60°C | 80°C |
| 0,5 % | 0,5 % | $5 \cdot 10^{-2}$ (8/+) | $1 \cdot 10^{-2}$ (9/+) | $6 \cdot 10^{-2}$ (8/+) |
| 0 % | 1 % | * - | - | - |
| | | Die Zahlen 8 und 9 geben die bei der Mikrokapillarentölung gemessenen Werte an; (+) bedeutet die Bildung einer Mittelphase | | |

* Lösungen nicht meßbar oder nicht wirksam

Beispiel 20

| | | Grenzflächenspannung (Spinning drop) $mNm^{-1}$ Lagerstättenöl I, 105/45 g/l $NaCl/CaCl_2$ | | | |
|---|---|---|---|---|---|
| Di-Styryl-o-kresol-6 EO-sulfonat Na | sec Alkan-sulfonat-Na | 25°C | 40°C | 60°C | 80°C |
| 0,5 % | 0,5 % | $10^{-1}$ (0/–) | $10^{-1}$ (0/–) | $10^{-2}$ (8/+) | $10^{-2}$ (6/+) |
| 0 % | 1,0 % | * - | - | - | - |
| | | Die Zahlen 0,6 bzw. 8 geben die bei der Mikrokapillarentölung gemessenen Werte an; (+) bzw. (–) bedeuten, daß sich eine Mittelphase gebildet hat bzw. nicht gebildet hat. | | | |

* Lösungen nicht meßbar oder nicht wirksam

Beispiel 21

| | | Grenzflächenspannung (Spinning drop) $mNm^{-1}$ Lagerstättenöl I, 105/45 g/l $NaCl/CaCl_2$ | |
|---|---|---|---|
| Mono-Styrol-p-kresol-8 EO-sulfonat Na | sec Alkan-sulfonat-Na | 60°C | 80°C |
| 0,5 % | 0,5 % | $4 \cdot 10^{-2}$ | $6 \cdot 10^{-2}$ |
| 0 % | 1 % | * - | - |

* Lösungen nicht meßbar oder nicht wirksam

Beispiel 22

| Di-Styryl-p-kresol-8 EO-sulfonat Na | sec Alkan-sulfonat-Na | Grenzflächenspannung (Spinning drop) $mNm^{-1}$ Lagerstättenöl I, 200 g NaCl 25°C | 40°C | 60°C | 80°C |
|---|---|---|---|---|---|
| 0,5 % | 0,5 % | $10^{-2}$ | $10^{-2}$ | $1 \cdot 10^{-2}$ (8/+) | $3 \cdot 10^{-2}$ (9/+) |
| 0 % | 1 % | * - | - | - | - |

Die Zahlen 8 bzw. 9 geben die bei der Mikrokapillarentölung gemessenen Werte an; (+) bedeutet die Bildung einer Mittelphase.

* Lösungen nicht meßbar oder nicht wirksam

Beispiel 23

| Di-Styryl-o-kresol-13 EO-sulfonat Na | Dodecylbenzol-sulfonat Na | Petrolsulfonat Na (MG 420) | Synergistische Tensidkombination 70/30 g/l NaCl/$CaCl_2$, Lagerstättenöl I, pH 6,5 (Grenzflächenspannung/Mikrokapillarentölung/Löslichkeit/Phasenverhalten) 25°C | 40°C | 60°C | 80°C |
|---|---|---|---|---|---|---|
| 0,7 % | 0,25 % | 0,05 % | lösl. (−) | lösl. (−) | lösl. (−) | lösl. (−) |
| 0,55 % | 0,40 % | 0,05 % | $5 \cdot 10^{-2}$ (6/+) | $4 \cdot 10^{-2}$ (8/+) | $6 \cdot 10^{-2}$ (9/+) | $4 \cdot 10^{-2}$ (9/+) |
| 0,50 % | 0,25 % | 0,25 % | lösl. (−) | lösl. (−) | lösl. (−) | lösl. (−) |
| 0,40 % | 0,45 % | 0,15 % | $3 \cdot 10^{-2}$ (6/+) | $4 \cdot 10^{-2}$ (9/+) | $4 \cdot 10^{-2}$ (9/+) | $6 \cdot 10^{-2}$ (9/+) |
| 0,30 % | 0,05 % | 0,05 % | $7 \cdot 10^{-3}$ (6/+) | $4 \cdot 10^{-2}$ (9/+) | $4 \cdot 10^{-2}$ (9/+) | $5 \cdot 10^{-2}$ (9/+) |

Die Zahlen 6,8 bzw. 9 geben die bei der Mikrokapillarentölung gemessenen Werte an, (+) bzw. (−) bedeuten, daß sich eine Mittelphase gebildet hat bzw. nicht gebildet hat.

Beispiel 24

| Di-Styryl-p-kresol-11 EO-sulfonat Na | sec Alkan-sulfonat Na | Petrolsul-sonat Na | Synergistische Tensidkombinationen 200 g/l NaCl Lagerstättenöl I, pH 6,5 (Grenzflächenspannung/ Mikrokapillarentölung/Löslichkeit/ Phasenverhalten) 40°C |
|---|---|---|---|
| 0,7 % | 0,25 % | 0,05 % | $8 \cdot 10^{-2}$ (3) |
| 0,45 % | 0,45 % | 0,1 % | $8 \cdot 10^{-3}$ (6) |
| 0,3 % | 0,65 % | 0,05 % | $4 \cdot 10^{-2}$ (3) |
| | | | Die Zahlen 3 bzw. 6 geben die bei der Mikrokapillarentölung gemessenen Werte an. |

Beispiel 25

| Di-Styryl-p-kresol-11 EO-sulfonat Na | sec Alkan-sulfonat Na | Alkylxylol-sulfonat Na | Synergistische Tensidkombinationen 200 g NaCl/l Lagerstättenöl I, pH 6,5 (Grenzflächenspannung/ Mikrokapillarentölung/Löslichkeit Phasenverhalten) 25°C | 40°C | 60°C | 80°C |
|---|---|---|---|---|---|---|
| | | | $1 \cdot 10^{-2}$ (6) | $9 \cdot 10^{-3}$ (6) | $9 \cdot 10^{-3}$ (6) | $1 \cdot 10^{-2}$ (6) |
| 0,4 % | 0,4 % | 0,2 % | $7 \cdot 10^{-3}$ (8) | $8 \cdot 10^{-3}$ (8) | $1 \cdot 10^{-2}$ (6) | $1 \cdot 10^{-2}$ (6) |
| | | | Die Zahlen 6 bzw. 8 geben die bei der Mikrokapillarentölung gemessenen Werte an | | | |

Beispiel 26

| Di-Styryl-o-kresol-13 EO-sulfonat Na | $C_{14}$–$C_{16}$-Olefin-sulfonat Na | Synergistische Tensidkombinationen 70/30 g/l NaCl/$CaI_2$, Lagerstättenöl I (Grenzflächenspannung/Mikrokapillarentölung/ Löslichkeit/Phasenverhalten) 40°C |
|---|---|---|
| 0,5 % | 0,5 % | $2 \cdot 10^{-2}$ $mNm^{-1}$ (6/+) |
| | | Die Zahl 6 gibt den bei der Mikrokappilarentölung gemessenen Wert an; (+) bedeutet die Bildung einer Mittelphase |

Die Effektivität der erfindungsgemäßen Verbindungen wurde durch Laborflutversuche mit Sandpackungen bestätigt.

Daten der Sandpackungen:
Sand: Korngröße0,03 - 0,15 mm
Länge15 cm
Durchmesser3 cm
Porenvolumen41 $cm^3$

Porosität48 %
Permeabilität800 mDasy
Haftwasser18 %
Oil in Place33,6 $cm^3$
Tensidslug20,5 $cm^3$; 2 % WS

Synth. Formationswasser:
Versuch 1 + 270 g NaCl/l + 30 g $CaCl_2$/l
Versuch 3 + 4200 g NaCl/l

Temperatur:80°C

Die Entölung nach einem Wasserfluten mit ca. der vierfachen Menge des Porenvolumens beträgt ca. 70 %. Das zurückbleibende Öl wird als Restölsättigung $S_R$ = 100 % angesetzt.
Die Mehrentölung ist in der anschließenden Tabelle wiedergegeben.

| Tensidsysteme | $S_R$ % |
|---|---|
| 1) 1,20 % Di-Styryl-o-kresol-8 EO-sulfonat Na/0,8 % sek. Alkansulfonat Na | 19,2 |
| 2) 0,8 % Di-Styryl-o-kresol-13 EO-sulfonat Na/1,1 % sek. Alkansulfonat Na/0,1 % Petrolsulfonat Na/1 % sek. Butanol | 45,3 |
| 3) 1,20 % Di-Styryl-p-kresol-11 EO-sulfonat Na/0,8 % sek. Alkansulfonat Na | 21,0 |
| 4) 1,07 % di-Styryl-p-kresol-11 EO-sulfonat Na/0,74 % sek. Alkansulfonat Na/0,19 % Petrolsulfonat Na | 31,6 |

**Patentansprüche**

1. Styrylaryloxy-ethersulfonat der Formel

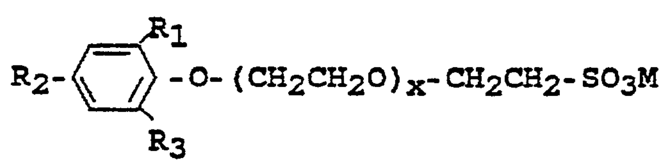

wobei entweder $R_1$ Styryl bedeutet und gleichzeitig $R_2$ und $R_3$ gleich oder verschieden sind und Wasserstoff oder Styryl bedeuten oder $R_1$ und $R_2$ ungleich sind und jeweils Methyl oder Styryl bedeuten und gleichzeitig $R_3$ Wasserstoff oder Styryl bedeutet, x eine Zahl von 2 bis 20 und M ein Ammonium- oder Alkalimetallkation bedeutet.

2. Styrylaryloxy-ethersulfonate nach Anspruch 1, wobei $R_1$ und $R_2$ ungleich sind und jeweils Methyl oder Styryl bedeuten und $R_3$ Wasserstoff oder Styryl bedeutet und x und M die in Anspruch 1 genannten Bedeutungen haben.

3. Verfahren zur Herstellung Styrylaryloxy -ethersulfonate nach Anspruch 1, dadurch gekennzeichnet, daß man eine Verbindung der Formel

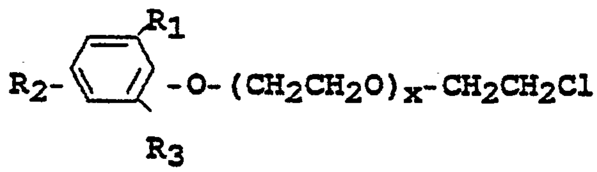

mit Alkalisulfit umsetzt.

4. Verwendung der Styrylaryloxy-ethersulfonate nach Anspruch 1 bei der Erdölförderung.

**Claims**

1. A styrylaryloxy ether sulfonate of the formula

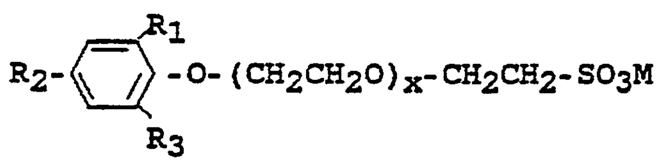

in which either $R_1$ denotes styryl and simultaneously $R_2$ and $R_3$ are identical or different and denote hydrogen or styryl, or $R_1$ and $R_2$ are nonidentical and each denote methyl or styryl and simultaneously $R_3$ denotes hydrogen or styryl, x denotes a number from 2 to 20, and M denotes an ammonium or alkali metal cation.

2. A styrylaryloxy ether sulfonate as claimed in claim 1, where $R_1$ and $R_2$ are nonidentical and each denote methyl or styryl, and $R_3$ denotes hydrogen or styryl, and x and M have the meanings mentioned in claim 1.

3. A process for the preparation of a styrylaryloxy ether sulfonate as claimed in claim 1, which comprises reacting a compound of the formula

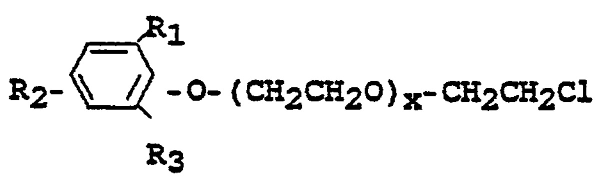

with an alkali metal sulfite.

4. The use of a styrylaryloxy ether sulfonate as claimed in claim 1 in oil recovery.

**Revendications**

1. Styrylaryloxysulfonate polyoxyéthyléné de formule

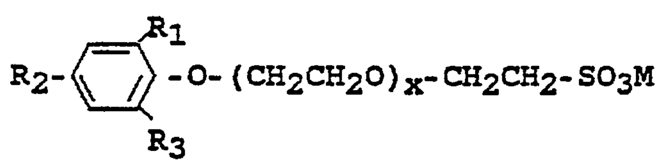

dans laquelle ou bien $R_1$ est le radical styryle et simultanément $R_2$ et $R_3$ sont identiques ou differents et représentent chacun un hydrogène ou le radical styryle, ou bien $R_1$ et $R_2$ sont différents et représentent chacun le radical méthyle ou styryle, et simultanément $R_3$ est un hydrogène ou le radical styryle, x est un nombre de 2 à 20 et M est un cation ammonium ou metal alcalin.

2. Styrylaryloxysulfonates polyoxyéthylénés selon la revendication 1, dans lesquels $R_1$ et $R_2$ sont différents et representent chacun le radical méthyle ou styryle, et $R_3$ est un hydrogène ou le radical styryle et x et M ont les significations données dans la revendication 1.

3. Procedé pour la preparation de styrylaryloxysulfonates polyoxyéthylénes selon la revendication 1, caractérisé en ce qu'on fait réagir avec le sulfite d'un metal alcalin un composé de formule

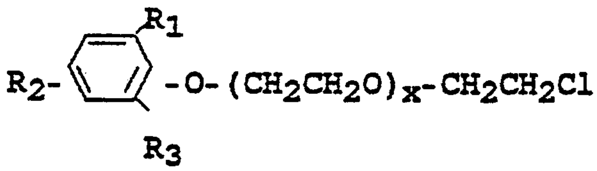

4. Utilisation des styrylaryloxysulfonates polyoxyéthylénés selon la revendication 1 pour la récupération du pétrole.